# EUROPÄISCHE PATENTSCHRIFT

(11) **EP 1 372 723 B1**
(45) Veröffentlichungstag und Bekanntmachung des Hinweises auf die Patenterteilung: **11.01.2006**
(21) Anmeldenummer: 02722195.1
(22) Anmeldetag: 08.03.2002
(51) Int. Cl.: A61K 39/44, A61K 48/00

(54) **SEMI-ALLOGENE ANTITUMOR-VAKZINE MIT HLA-HAPLO-IDENTISCHEN ANTIGEN-PRÄSENTIERENDEN ZELLEN**
SEMI-ALLOGENIC ANTI-TUMOUR VACCINE WITH HLA HAPLO-IDENTICAL ANTIGEN-PRESENTING CELLS
VACCINS ANTI-TUMORAUX SEMI-ALLOGENES COMPORTANT DES CELLULES PRESENTANT DES ANTIGENES HLA HAPLO-IDENTIQUES

(30) Priorität: 16.03.2001 DE 10112851
(43) Veröffentlichungstag der Anmeldung: 02.01.2004
(73) Patentinhaber: GSF-Forschungszentrum für Umwelt und Gesundheit GmbH, 85764 Oberschleissheim (DE)
(72) Erfinder: SCHENDEL, Dolores, 80469 München (DE); WANK, Rudolf, 80469 München (DE)
(74) Vertreter: Behnisch, Werner
(86) Internationale Anmeldenummer: PCT/EP2002/002595
(87) Internationale Veröffentlichungsnummer: WO 2002/074338

(56) Entgegenhaltungen:
- WO-A-00/76537
- WO-A-98/33527
- US-A- 6 063 375

## Beschreibung

Die vorliegende Erfindung betrifft semi-allogene Antigen-präsentierende Zellen, in welche Proteine und/oder Peptide oder RNA bzw. DNA oder cDNA, die für diese Proteine und/oder Peptide codieren, die in Tumorzellen überexprimiert werden oder die aus Tumorzellen stammen, eingebracht sind Die Erfindung betrifft weiterhin Verfahren zur Erzeugung dieser semi-allogenen Antigen-präsentierenden Zellen sowie deren Verwendung zur Behandlung von Tumorerkrankungen.

Drei kürzlich erfolgte Entwicklungen legen nahe, daß zellvermittelte Immunreaktionen zu einer beträchtlichen Verminderung der Tumorbelastung und zu einer ebenso beträchtlichen Verlängerung der Überlebenszeit der Patienten bei Tumorerkrankungen, wie beispielsweise einem metastasierenden Nierenzellkarzinom (Metastatic Renal Cell Carcinoma = mRCC), führen können. Klinische Daten haben bereits Erfolge für mRCC gezeigt, jedoch ist auch eine Übertragung derselben Prinzipien auf andere Tumoren denkbar. Im Folgenden werden drei Entwicklungen beschrieben, die den Ausgangspunkt für den erfindungsgemäßen Ansatz bilden.

### I. Allogene Knochenmarkstransplantation zur Behandlung des metastasierenden Nierenzellkarzinoms

Nierenzellkarzinom-Patienten, die einer allogenen Knochenmarkstransplantation unterworfen werden, können in 53 % der Fälle eine Voll- oder Teil-Remission erreichen (1). Die Lebenserwartung kann beträchtlich von einigen Monaten auf mehrere Jahre erhöht werden. Während bedeutende Tumorbelastungen beseitigt werden können, entwickeln 53 % der Patienten die Graft-versus-host-Krankheit (GVHD), die mit einer GVHD-assoziierten Sterblichkeit von 5 % verbunden ist.

Der Mechanismus der Tumorrückbildung ist vermutlich mit dem vergleichbar, der mit der Eliminierung hämatologischer Malignome im Anschluß an eine allogene Knochenmarkstransplantation (BMT) verbunden ist (2). Aktivierte T-Lymphozyten erkennen Liganden, die aus Peptiden, die von einem in Tumorzellen exprimierten Protein abstammen, und MHC-Molekülen (sowohl des Klasse I- als auch des Klasse II-Typs) bestehen. Die MHC-Proteine werden in drei Gruppen eingeteilt, von denen zwei Gruppen, nämlich die MHC-Proteine der Klasse I und II, in der vorliegenden Erfindung von besonderer Bedeutung sind. Bei Menschen werden die MHC-Klasse I-Proteine als HLA-A, -B, -C bezeichnet. Sie werden auf fast allen Körperzellen exprimiert und stellen starke Transplantationsantigene mit hochgradigem Polymorphismus dar. Die MHC-Klasse II-Proteine beim Menschen sind HLA-DP, -DQ, -DR und werden beispielsweise auf dendritischen Zellen und stimulierten Makrophagen exprimiert und stellen ebenfalls Transplantationsantigene mit hochgradigem Polymorphismus dar.

Die Erkennung der Liganden aktiviert in verschiedenen T-Zellen Effektorfunktionen (beispielsweise Cytokin-Sezernierung und Zytotoxizität), wodurch eine Zerstörung von Tumorzellen erfolgt Die parallele Aktivierung von T-Zellen, die Liganden aus MHC und nicht Tumor-assoziierten Peptiden erkennen (d.h. Alloreaktionen), kann durch Sezemierung von Entzündungs-Cytokinen, die wiederum Antitumor-Reaktionen unterstützen, zu einer optimalen Immunreaktion beitragen. Die Reaktionen dieser Zellen spielen jedoch ebenfalls eine Rolle bei der GVHD.

### II. Hybrid-Vakzine unter Verwendung von autologen Tumorzellen und allogenen dendritischen Zellen (DC)

Kugler et al. (3, 4) haben gezeigt, daß mRCC-Patienten mit Hybrid-Vakzinen "vakziniert" werden können, die aus fusionierten allogenen dendritischen Zellen und autologen TumorZellen von nichtverwandten Individuen bestehen. Im Anschluß an die "Vakzinierung" zeigen die Patienten eine deutliche Abnahme der Tumorbelastung. Weniger Patienten erreichen eine vollständige Remission im Vergleich zu Ansatz I, eine gesteigerte Lebenserwartung wurde bis jetzt nicht festgestellt. Im Gegensatz zur Behandlung bei Ansatz I zeigt der Hybrid-Vakzin-Ansatz jedoch keine oder nur geringe Toxizität Einige Tumor-Patienten erlitten nach dem Ende der Vakzinierung einen Rückfall, der vermutlich auf die fehlende Aufrechterhaltung von Langzeit-T-Zell-Reaktionen zurückzuführen ist. Dieses Problem kann durch kontinuierliche Vakzinierung eventuell überwunden werden, jedoch ist die Vakzinierung durch die Verfügbarkeit von Tumorzellen beschränkt, die als Fusionspartner für die allogenen dendritischen Zellen dienen. Somit ist keine weitere Behandlung möglich, wenn die Tumorzellen zur Neige gehen.

### III. Aus Tumorzellen gewonnene RNA zur Erzeugung von Vakzinen auf Grundlage von autologen DC's

Gilboa und Mitarbeiter (5-8) haben eine Strategie zum Primen von autologen DCs mit autologer, aus Tumorzellen gewonnener RNA entwickelt, die von DCs aufgenommen, in ein Protein translatiert und zu Peptiden zur Präsentation mit autologen MHC Klasse I und Klasse II-Molekülen verarbeitet werden kann. Um eine unbegrenzte Quelle an Material zum DC-Pulsen bereitzustellen, wird RNA aus Tumorzellen zu cDNA rücktranskribiert, die dann als eine Matrize zur in vitro-Transkription weiterer RNA dient. Eine PCR-Amplifizierung ermöglicht die Erzeugung von unbeschränktem Material. Die Expression von aus Tumoren gewonnener RNA in autologen DCs ermöglicht, daß MHC-Peptid-Liganden an der DC-Zelloberfläche exprimiert werden, die denjenigen entsprechen, die auf der Tumorzell-Oberfläche zu finden sind. Die co-stimulatorischen Moleküle der DC und die Cytokine, die sie produziert, ermöglichen eine optimale Stimulierung von T-Zell-Reaktionen. Diese Vakzine zeigen keine oder nur geringe Toxizität, jedoch wurde bis zum heutigen Tage keine mit I und II vergleichbare Rückbildung publiziert. Dies kann auf das Fehlen von allo-responsiven T-Zellen zurückzuführen sein, die in einem autologen System nicht aktiviert sind

Zusammenfassend bleibt festzustellen, daß die konventionellen Therapieansätze unter beträchtlichen Nachteilen leiden, die einen dauerhaften therapeutischen Erfolg verhindern oder schwerwiegende unerwünschte Wirkungen mit sich bringen.

So leidet, wie bereits oben dargelegt, der Ansatz I unter dem Risiko der Entstehung der GVHD, die in 53 % der Fälle auftritt und teilweise mit einem tödlichem Ausgang verbunden ist.

Der unter II beschriebene Ansatz (Kugler et al.) weist den Nachteil auf, daß lebensfähige Tumorzellen des Patienten benötigt werden, um die MHC-Peptid-Liganden bereitzustellen und ist somit in seiner Anwendungsdauer stark eingeschränkt.

Die Therapie von Gilboa und Mitarbeiter weist zwar nur geringe Toxizität auf, es wurde jedoch bis jetzt keine nennenswerte Tumorrückbildung publiziert.

Es ist daher die Aufgabe der vorliegenden Erfindung, Antigen-präsentierende Zellen bereit zu stellen, die die vorstehenden Nachteile vermeiden und so in der Therapie von Tumorerkrankungen verwendbar sind.

Diese Aufgabe wird durch die in den unabhängigen Ansprüchen dargelegten Merkmale gelöst. Bevorzugte Ausführungsformen sind in den abhängigen Ansprüchen angegeben.

Durch Einsatz von semi-allogenen Antigen-präsentierenden Zellen, in die Proteine und/oder Peptide oder RNA bzw. DNA oder cDNA, die für diese Proteine und/oder Peptide codieren, die in Tumorzellen überexprimiert werden oder die aus autologen Tumorzellen oder aus mehreren unterschiedlichen Tumorzell-Linien stammen, eingebracht wurden, kann eine für die Behandlung von Tumorerkrankungen vorteilhafte Immunreaktion erreicht werden.

Das erfindungsgemäße Verfahren zur Erzeugung dieser semi-allogenen Antigen-präsentierenden Zellen weist, anders als der Ansatz von Kugler et al., den Vorteil auf, daß keine Elektrofusion erforderlich ist. Die Funktion der Antigen-präsentierenden Zellen wird daher nicht durch Elektrofusion gestört. Eine Elektrofusion kann deswegen vermieden werden, weil die Transfektion beispielsweise mit RNA die natürliche Fähigkeit von Antigen-präsentierenden Zellen ausnutzt, derartige Moleküle aufzunehmen.

Die erfindungsgemäße Strategie, d.h. der Einsatz einer semi-allogenen Vakzine, bringt eine erhöhte Effizienz der Präsentation von Liganden bestehend aus MHC und Tumor-assoziiertem Peptid mit sich. Nur ungefähr 10% Hybridzellen werden bei dem Ansatz von Kugler et al. erzeugt, wohingegen erfindungsgemäß ein wesentlich höherer Prozentsatz an Antigen-präsentierenden Zellen, die Peptid-Liganden exprimieren, erzeugt werden kann.

Dabei ist der gemäß einer bevorzugten Ausführungsform zur (unbegrenzten) Erzeugung von Tumor-assoziierter RNA verwendete Ansatz besonders vorteilhaft, mit dem z. B. RNA aus autologen Tumorzellen in cDNA rücktranskribiert, die cDNA mit Hilfe der PCR amplifiziert und anschließend die cDNA in RNA transkribiert wird.
Dieser erfindungsgemäße Ansatz weist den Vorteil auf, daß bereits kleine Mengen von Tumorzellen ausreichend RNA liefern können, um eine unbegrenzte cDNA-Quelle für die in vitro RNA-Transkription zu bilden. Ein Pulsen auf RNA-Grundlage ermöglicht es daher, die mit der beschränkten Anzahl von Tumorzellen einhergehenden Nachteile zu vermeiden. Dauerhafte Möglichkeiten, Vakzinierungen durchzuführen, verbessern die Reaktionsgeschwindigkeiten und deren Langlebigkeit im Vergleich mit beispielsweise dem Ansatz von Kugler et al.

Die den MHC-Molekülen der Patienten nicht entsprechenden MHC-Moleküle von semi-allogenen Antigen-präsentierenden Zellen stellen einen Stimulus für Allo-Reaktionen dar. Durch Einsatz semi-allogener Antigen-präsentierender Zellen wird daher im Vergleich zur Verwendung autologer Zellen eine verstärkte Immunreaktion hervorgerufen. Trotzdem ist die Toxizität der erfindungsgemäßen Vakzine weit geringer als bei den bisher beschriebenen Ansätzen.

Die erfindungsgemäße Verwendung von semi-allogenen dendritischen Zellen stellt so das notwendige MHC-Matching des Ansatzes von Gilboa et al. bereit, vermeidet jedoch den mit dem Einsatz von autologen dendritischen Zellen einhergehenden Nachteil einer schwachen Funktion der autologen dendritischen Zellen, die oftmals mit einer fortgeschrittenen Erkrankung bei Krebspatienten verbunden ist

Erfindungsgemäß werden Antigen-präsentierende Zellen aus Individuen präpariert, die bezüglich des Patienten semi-allogen sind. Dies ist dann der Fall, wenn die MHC-Moleküle von Antigen-präsentierenden Zellen eines verwandten Spenders, beispielsweise von Vater, Mutter, Geschwistern oder Kindern, den MHC-Molekülen des Patienten zu 50% entsprechen. Dies kann schematisch wie folgt dargestellt werden: Wenn das Patienten-HLA die Allele a+c aufweist, müssen Familienangehörige als potentielle Spender folgende Allel-Varianten aufweisen:
- Eltern:: HLA-ab oder cd
- Geschwister:: HLA-ad oder bc
- Kinder:: HLA-ae, af, ce, cf
(Mutter = HLA-ef)

Die vorgenannten semi-allogenen Antigen-präsentierenden Zellen sind insofern bezüglich denjenigen des Patienten HLA-haploidentisch und können deswegen auch als HLA-haploidentische Antigen-präsentierende Zellen bezeichnet werden.

HLA-haploidentische Antigen-präsentierende Zellen teilen Klasse I-(HLA-A, B und C) Moleküle mit dem Patienten, die von den HLA-A, B und C-Allelen eines Chromosoms codiert werden. Sie teilen ebenfalls Klasse II-Moleküle (BLA-DR, DQ und DP) mit dem Patienten, die durch die entsprechenden Allele desselben Chromosoms codiert werden.

Nach der Isolierung der semi-allogenen Antigen-präsentierenden Zellen werden in diese Proteine und/oder Peptide oder RNA bzw. DNA oder cDNA oder cRNA, die für diese Proteine und/oder Peptide codieren, aus autologen Tumorzellen eingebracht. In einer weiteren Ausführungsform werden Proteine und/oder Peptide oder RNA, DNA, cDNA oder cRNA, die für diese Proteine und/oder Peptide kodieren, eingebracht, die in Tumorzellen überexprimiert werden. Dabei handelt es sich um tumordefinierte Antigene, d. h. Antigene, die für Tumorzellen spezifisch sind und in den Tumorzellen stärker exprimiert werden bzw. deutlich stärker exprimiert werden als in Normalzellen. Bevorzugt werden derartige tumordefinierte Antigene ausgewählt aus Onkogenen, beispielsweise HBR2/neu, Proteinen, die dem Tumor einen Wachstumsvorteil verschaffen und/oder sein Überleben sichern, z. B. PSMA, Zellzyklusregulationsproteinen, Transkriptionsfaktoren, z. B. WT-1, Muci nen, bevorzugt MUC1, Proteinen, die in der Regulation der Zellteilung involviert sind, bevorzugt Telomerase. Es versteht sich von selbst, daß die hier genannten Proteine, Peptide, RNA, DNA, cDNA und cRNA auch rekombinant herstellbar und einsetzbar sind

Als Folge präsentieren die Antigen-präsentierenden Zellen einen Liganden aus MHC und Tumor-assoziiertem Peptid, der von T-Zellen erkannt wird und daher eine gegen den autologen Tumor gerichtete Immunreaktion auslöst.

Gemäß einer bevorzugten Ausführungsform ist die Verwendung von Antigen-präsentierenden Zellen zweier unterschiedlicher semi-allogener Spender (d.h. beispielsweise Mutter und Vater) vorgesehen, so daß die durch die semi-allogenen DC's präsentierten MHC's alle potentiellen MHC Klasse I und Klasse II-Moleküle des Patienten umfassen. Das zweite Chromosom jedes Spenders codiert zusätzlich allogene MHC-Moleküle, die zur Induktion von Allo-Reaktionen dienen können.

Um MHC Klasse I und Klasse II-Peptid-Liganden bereitzustellen, die denjenigen der Patienten-Tumorzellen entsprechen, wird die bereits bekannte Strategie von Gilboa und Mitarbeiter angewendet, um eine unbegrenzte Quelle von aus Tumoren gewonnener RNA zum Pulsen Antigen-präsentierender Zellen herzustellen. Eine gleichwertige Möglichkeit besteht darin, die semi-allogenen Antigen-präsentierenden Zellen mit anderen Antigen-Quellen, wie beispielsweise Peptiden und Proteinen, zu pulsen (9).

Gemäß einer Ausführungsform der Erfindung stammen die Proteine und/oder Peptide oder RNA bzw. DNA, cDNA oder cRNA die für diese Proteine und/oder Peptide codieren, die in Tumorzellen überexprimiert werden oder die aus autologen Tumorzellen stammen, aus Tumorzellen, bevorzugt autologen Tumorzellen bzw. werden in diesen Tumorzellen überexprimiert, wobei die Tumorzellen umfassen: Zellen aus mesenchymalen Tumoren, z. B. Zellen aus Sarkomen, Karzinomen, bevorzugt Ovarial-, Mamma- und Nierenzellkarzinomen, Tumorzellen des blutbildenden Systems, bevorzugt Zellen aus Leukämien und Lymphomen, Zellen aus epithelealen Tumoren, Zellen aus ektodermalen Tumoren, z. B. Melanome, und Zellen aus embryonalen Tumoren aus undifferenziertem Gewebe, bevorzugt Blastome und Teratome. Ein Beispiel ist das hier näher untersuchte Nierenzellkarzinom, insbesondere das metastatische Nierenzellkarzinom. Die semi-allogenen Antigen-präsentierenden Zellen der Erfindung können dann zur Behandlung der Tumoren eingesetzt werden.

Wie bereits oben angesprochen, kann die zur Aufnahme in die Antigen-präsentierenden Zellen vorgesehene RNA aus autologen Tumorzellen zur unbegrenzten Vermehrung in cDNA rücktranskribiert, die cDNA mit Hilfe der PCR amplifiziert und anschließend die cDNA in RNA transkribiert wird

Die Verabreichung der erfindungsgemäßen Antigen-präsentierenden Zellen erfolgt intravenös, subkutan oder intramuskulär, wobei eine subkutane Injektion bevorzugt wird.

Zur Verwendung in der vorliegenden Erfindung sind alle Arten von Antigen-präsentierenden Zellen einsetzbar. Diese schließen insbesondere Monocyten/Makrophagen und dendritische Zellen ein, wobei Letztere bevorzugt werden.

Ein weiterer Aspekt der vorliegenden Erfindung besteht darin, anstatt der Bestandteile autologer Tumorzellen einen Pool von Bestandteilen mehrerer unterschiedlicher Tumorzell-Linien einzusetzen, der in die semi-allogenen Antigen-präsentierenden Zellen eingebracht wird. Diese können dann als Vakzine in der Tumorbehandlung eingesetzt werden. Um eine derartige "generische" Vakzine zu bilden ist es vorteilhaft, beispielsweise einen Pool von cDNA, wie oben beschrieben, von 3-5 unterschiedlichen RCCs herzustellen, der dann für alle Patienten verwendet werden kann. Denn es ist davon auszugehen, daß die meisten tumorspezifischen T-Zellen, die gegen derartige Pool-gepulste Antigen-präsentierende Zellen geprimed werden, autologe Tumorzellen aufgrund der Tatsache erkennen können, daß sie gemeinsame Peptid-MHC-Liganden aufweisen. Dies bedeutet, daß es ausreicht, ein einziges Produkt (d.h. eine generische cDNA und ihre entsprechende in vitro transkribierte cRNA) unter GMP-Bedingungen (GMP = Good Manufacturing Practice) zu erzeugen. Dann müßen nur noch die semi-allogenen dendritischen Zellen für jeden Patienten, der mit der erfindungsgemäßen Vakzin-Therapie behandelt werden soll, unter GMP-Bedingungen präpariert werden.

Die vorliegende Erfindung umfasst zuletzt eine pharmazeutische Zusammensetzung, die die oben beschriebenen semi-allogenen Antigen-präsentierenden Zellen enthält, wobei die Zusammensetzung vorzugsweise in Form einer Vakzine vorliegt. In den Abbildungen zeigen:

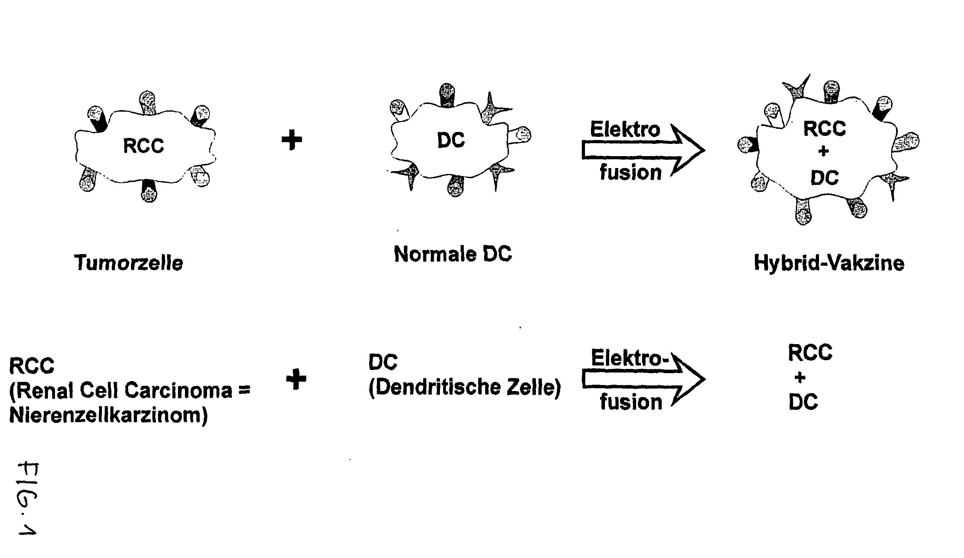

- Abb.1: das Prinzip der Erzeugung einer allogenen Tumorvakzine nach Kugler et al., bei dem eine autologe Tumorzelle und eine allogene dendritische Zelle von nichtverwandten Individuen fusioniert werden,
- Abb.2: den erfindungsgemäßen Ansatz, bei dem eine semi-allogene Vakzine unter Verwendung von HLA-haploidentischen Antigen-präsentierenden Zellen erzeugt wird.

Die haploidentische Vakzine weist Folgendes auf:
Liganden aus MHC und TAA-(Tumor-assoziiertem Antigen-) Peptid für die Hälfte der Patienten-Allele.
Alle Allele können unter Verwendung von zwei haploidentischen DC-Zubereitungen gematcht werden
HLA-misgematchte MHC-Peptid-Liganden zur Stimulierung von Alloreaktionen und von T-Helferzellen
Von DC's stammende co-stimulatorische Moleküle und Cytokine zur optimalen Unterstützung der Immunreaktionen (diese sind in der Abb. als "Y" wiedergegeben)

Im Folgenden werden die Grundgedanken der Erfindung sowie Testmodelle zu deren Veranschaulichung am Beispiel von dendritischen Zellen ausführlicher beschrieben.

Es werden zwei Modellsysteme verwendet, um die Prinzipien dieses Ansatzes zu verdeutlichen. Das erste verwendet einen gut charakterisierten Satz an Reagenzien aus einem Melanom-Modell, das zweite einen Satz an Reagenzien aus einem Nierenzellkarzinom-Modell. Das Melanommodell weist den Vorteil auf, daß das vom T-Zellrezeptor (TCR) erkannte Epitop (MHC-Peptid Ligand) des Testklones (Tyr-F8) identifiziert wurde; dies macht es möglich, die Effizienz der Antigen-Präsentation durch Antigen-präsentierende Zellen, wie beispielsweise dendritische Zellen, in einer quantitativeren Weise zu untersuchen. Das Epitop für einen Nierenzellkarzinom-spezifischen T-Zell-Klon (TIL-26) wurde bis jetzt nicht identifiziert, jedoch verwenden diese zytotoxischen T-Lymphozyten (CTL) hochcharakteristische TCR, die identifiziert und in vitro mit dem Verfahren der Echtzeit RT-PCR auf Grundlage der Molekularsequenz der TCR CDR3-Region quantifiziert werden können. Dies ermöglicht es, die funktionelle Priming-Kapazität der dendritischen Zellen zu identifizieren, indem die T-Zellen durch diese charakteristischen TCR-Sequenzen identifiziert werden. In Kombination können diese beiden Modelle verwendet werden, um die Spezifität und die Effizienz dieses Systems zum Präsentieren von tumorassoziierten Peptiden auf der Oberfläche von beispielsweise dendritischen Zellen und zur Aktivierung von spezifischen T-Zell-Reaktionen in vitro zu demonstrieren. Diese essentiellen Experimente beweisen noch vor Einleitung der klinischen Phase I/II das Funktionieren des der Erfindung zu Grunde liegenden Prinzips.

### Melanomsystem

Die Reagenzien für dieses Modellsystem wurden von Professor Peter Schrier (Universität Leiden) bezogen. Ein HLA-A*0201-restringierter CTL-Klon (Tyr F8) wurde von einem Patienten mit fortgeschrittenem Melanom isoliert. Eine autologe Melanom-Tumorzell-Linie wurde ebenfalls von dem Patienten isoliert Der spezifische Ligand, d.h. das aus einem Tumor-assoziierten Protein gewonnene Peptid, ist bekannt; es wird aus Tyrosinase, einem Enzym, das in gesunden Melanozyten in normalem Spiegel exprimiert wird, das jedoch in Melanom-Zellen überexprimiertwird, gewonnen. Das präsentierende MHC-Molekül wurde als von dem HLA-A*0201-Allel codiert identifiziert. Die cDNA für Tyrosinase ist verfügbar; das exakte, aus dem Tyrosinase-Protein gewonnene neun-Aminosäure-Peptid, das auf der Peptid-Bindungs-Rille des HLA-A2-Moleküls vorliegt, ist bekannt und der Tyr F8 T-Zell-Klon ist verfügbar (10).

Der allgemeine Ansatz des Experiments besteht zunächst darin, RNA aus der Melanom-Linie zu präparieren und dann cDNA durch reverse Transkription herzustellen. Diese cDNA wird dann amplifiziert und als eine Matrize zur Herstellung von in vitro transkribierter RNA (cRNA) verwendet. Die cRNA wird zum Pulsen von HLA-A2-positiven dendritischen Zellen (11, 12) verwendet. Diese dendritischen Zellen werden die cRNA aufnehmen, und sie dann in Proteine translatieren, wovon ein Protein das Tyrosinase-Enzym sein sollte. Das Tyrosin-Protein wird intrazellulär in den dendritischen Zellen zu Peptid-Bruchstücken verarbeitet und das spezifische neun-Aminosäure-Epitop wird im endoplasmatischen Retikulum der dendritischen Zellen auf neu synthetisierte HLA-A2-Moleküle aufgeladen. Diese MHC-Peptid-Komplexe werden dann zur Plasmamembran der dendritischen Zelle transportiert, wo sie durch T-Zellen erkannt werden können. Tyr F8-Zellen, die den geeigneten MHC-Peptid-Komplex auf der DC-Oberfläche erkennen, reagieren durch Sezernieren von Interferon-Gamma und durch Aktivieren des zytolytischen Mechanismus, der es ihnen ermöglicht, die dendritische Zelle abzutöten. Diese Aktivierung kann durch eine ELISA-Detektion der Interferon-Gamma-Sezernierung durch den Tyr F8-Klon oder durch Verwendung eines Standard-Chrom-Freisetzung-Assay bestimmt werden, indem die dendritischen Zellen als Zielzellen markiert werden und indem ihre Lysis in Gegenwart von Tyr F8-Zellen gemessen wird. Die Aktivierung der Tyr F8-Zellen ist sehr spezifisch, so daß MHC-Moleküle, die von anderen Proteinen als Tyrosinase gewonnene Peptide tragen, die Tyr F8-Zellen nicht aktivieren können und ein bestimmter Grenzwert dieser spezifischen MHC-Tyrosinase-Peptid-Komplexe muß durch die DC präsentiert werden, um die Tyr F8-Zellen optimal zu stimulieren.

Um zu bestimmen, ob die originale cRNA das notwendige Startmaterial bereitstellen kann, um der dendritischen Zelle die Ausführung all dieser Schritte zu ermöglichen, werden cRNA gepulste dendritische Zellen als stimulierende Zellen zur Aktivierung der Tyr F8-Zellen verwendet. Weil die Melanomtumorzellen eine Vielzahl unterschiedlicher RNAs liefern, repräsentieren Transkripte für Tyrosinase nur einen Bestandteil eines viel größeren Pools. Die Fähigkeit dieses gemischten cRNA-Pools zur Bereitstellung des Tyrosinase-Epitops kann mit cRNA verglichen werden, die aus der isolierten Tyrosinase cDNA (d.h. nur eine RNA-Spezies) hergestellt wurde. Eine relative Beurteilung des Spiegels an HLA-A2-Tyrosinase-Peptid-Komplexen kann durch Pulsen von dendritischen Zellen mit variierenden Mengen an Test-cRNA und durch einen Vergleich derer stimulatorischen Fähigkeiten mit dendritischen Zellen erfolgen, die mit unterschiedlichen bekannten Mengen eines synthetischen Peptids gepulst wurde, das das neun-Aminosäure-Epitop von Tyrosinase darstellt. Der Grenzwert, bei dem die Tyr F8-Zellen nicht mehr länger den korrekten MHC-Peptid-Komplex nachweisen können, kann durch Bestimmung der von Tyr F8-Zellen freigesetzten Menge an Interferon-Gamma unter Verwendung eines spezifischen ELISA-Assays nachgewiesen werden.

### Nierenzellkarzinom-System

Diese Reagenzien wurden von den Erfindern erzeugt. Eine Nierenzellkatzinom-spezifische CTL-Linie wurde von einem Nierenzellkarzinom-Patienten isoliert und T-Zell-Klone wurden aus dieser T-Zell-Linie gewonnen (TIL-26). Autologe Tumorzellen wurden ebenfalls von diesem Patienten gewonnen (RCC-26). Das MHC-Restriktionsmolekül, das ein Tumor-assoziiertes Peptid präsentiert und das von RCC-26-Zellen dargeboten wurde, wurde als das von HLA-A*0201-codierte Molekül identifiziert. Wenn die TIL-26-Zellen den autologen RCC-26 Tumorzellen ausgesetzt wurden, wurden sie zum Sezernieren von Interferon-Gamma aktiviert und sie waren dazu in der Lage, die autologen RCC-26 Tumorzellen zu lysieren (13).

Der allgemeine Entwurf dieses Experiments ist dem für das Melanom-System beschriebenen ähnlich. Im ersten Schritt wird RNA aus RCC-26 Zellen hergestellt und cDNA wird durch reverse Transkription hergestellt. Diese dient als die Matrize zur Erzeugung von cRNA durch in vitro Transkription. Die cRNA wird zum Pulsen der dendritischen Zellen verwendet und diese dendritischen Zellen werden auf ihre Fähigkeit hin überprüft, die TIL-26-Zellen zur Sekretion von Interferon-Gamma zu aktivieren. Die dendritischen Zellen werden ebenfalls im Anschluß an das Pulsen mit cRNA, die von RCC-26 Zellen stammte, in zytotoxischen Assays unter Verwendung von aktivierten TIL-26 Zellen als Zielzellen beurteilt. Die Effizienz der Stimulierung der dendritischen Zellen kann mit derjenigen der RCC-26 Zellen bei Assays verglichen werden, die die Interferon-Gamma-Sekretion durch die TIL-26 Zellen messen und als Zielzellen in Zytotoxizitäts-Assays.

Diese beiden Experimentreihen zeigen deutlich, daß die Verfahren zur Erzeugung von MHC-Peptid-Komplexen unter Verwendung einer cRNA-gepulsten allogenen (HLA-A*0201) dendritischen Zelle funktionieren.

### Induktion von T-Zell-Reaktionen durch cRNA-gepulste allogene dendritische Zellen:

Um die Fähigkeit der cRNA-gepulsten dendritischen Zellen zum Primen von allogenen T-Zellen zu beurteilen, werden zwei Experimente in vitro durchgeführt. Zuerst wurde gezeigt, daß naive T-Zellen aus Spender DS (DS = anonymer Spender) in vitro mit RCC-26 Tumorzellen geprimed werden können, um ein Epitop zu erkennen, das offensichtlich dasselbe ist, wie das von TIL-26 (14) erkannte Epitop. Wie TIL-26 Zellen exprimieren die T-Zellen von Spender DS TCRα-Ketten, die zu den von TIL-26 Zellen exprimierten (15) hochgradig homolog sind. Die TCR-Sequenz dieser T-Zellen kann durch Isolierung von RNA aus einer Testprobe und unter Verwendung von TCR-spezifischen Primern zur Analyse der Transkriptmenge in der Probe unter Verwendung des Verfahrens der Echtzeit RT-PCR (14) quantifiziert werden. Zuerst werden allogene HLA-A*0201-positive dendritische Zellen, die mit cRNA (aus RCC-26 gewonnen) gepulst werden, als stimulierende Zellen in gemischten Lymphozyten-Kulturen unter Verwendung peripherer Blut-Lymphozyten (PBL) von Spender DS als reagierende Zellen verwendet. Das Auftreten der Marker (Va20) T-Zellen, die das auch von TIL-26 Zellen erkannte Epitop erkennen, wird überwacht, indem nach Zunahmen von Transkcipten mit charakteristischen Va20-Sequenzen gesucht wird Eine quantitative Bestimmung kann unter Verwendung einer Echtzeit RT-PCR durchgeführt werden, die die Häufigkeit derartiger Transkripte in den unstimulierten PBL und im Anschluß an eine Stimulierung mit den cRNA-gepulsten dendritischen Zellen in vitro nach verschiedenen Umläufen der Restimulation vergleicht. Autologe dendritische Zellen, die aus Spender DS gewonnen wurden, die mit cRNA (RCC-26-gewonnen) gepulst sind, werden als Zielzellen verwendet und direkt mit RCC-26 Tumorzellen verglichen, indem als zytotoxische Effektorzellen die mit cRNA-gepulsten dendritischen Zellen geprimten PBL versus mit intakten RCC-26 Tumorzellen geprimte PBL verwendet werden.

Als zweites Testsystem werden T-Zellen von einem HLA-A*0201-Spender mit cRNA-gepulsten (aus einem Melanom gewonnenen) dendritischen Zellen stimuliert, die von einem Spender gewonnen wurden, der HLA-A*0201 in gemischten Lymphozytendendritischen Zell-Kulturen exprimiert. Die Entwicklung von T-Zellen, die den Tyrosinspezifischen Liganden erkennen, wird durch Standarddurchflußzytometrie unter Verwendung von HLA-A2-Tyrosinase-Peptid-spezifischen Tetrameren (16) beurteilt.

### Pulsen von dendritischen Zellen mit gemischten cRNA-Proben:

Es wird gegenwärtig angenommen, daß Immunreaktionen, die gegen multiple Epitope erzeugt werden, die beste Möglichkeit zur Eliminierung von Tumorzellen und zum Schutz gegen einen Tumor-Rückfall darstellen. Dies basiert auf der Beobachtungen, daß in vivo gegen individuelle MHC-Peptid-Liganden geprimte CTL zur Erzeugung von CTL geführt haben, die für den MHC-Peptid-Liganden spezifisch sind und in einigen Fällen zur Tumor-Rückentwicklung geführt haben. Es können jedoch Tumorvarianten entstehen, die nicht mehr länger die MHC-Moleküle exprimieren und nicht mehr länger das Protein exprimieren, aus dem das spezifische Peptid gewonnen wird. Wenn CTL's mit Spezifitäten für viele unterschiedliche MHC-Peptid-Liganden erzeugt werden, dann ist die Entstehung von Tumorvarianten in geringerem Maße möglich, weil individuelle Tumorzellen unterschiedliche Mutationen in allen MHC-Molekülen und Proteinen, die Peptide bereitstellen, exprimieren müßten, um der Erkennung durch CTL mit vielen unterschiedlichen Spezifitäten zu entgehen.

Es ist deswegen optimal, cRNA aus mehreren Tumorzell-Linien zu poolen, um viele unterschiedliche Tumor-assoziierte Peptide für einen Immunangriff bereitzustellen. Ähnlich ist es auch besser, mehrere unterschiedliche MHC-Moleküle zu verwenden, die von den reagierenden T-Zellen und den stimulierenden dendritischen Zellen geteilt werden. Dies wird in der hier beschriebenen Strategie erreicht, indem HLA-haplo-identische dendritische Zellen, die von Familienmitgliedern des Spenders der T-Zellen gewonnen werden, wie unten beschrieben verwendet werden. Dabei muß jedoch sichergestellt werden, daß durch Poolen von cRNA aus unterschiedlichen Tumoren die erforderliche Dichte an MHC-Peptid-Liganden, die für spezielle Epitope auf einer Tumorzell-Linie charakteristisch sind, noch erhalten bleibt Dies kann experimentell erreicht werden, indem cRNA aus der Melanomlinie mit cRNA aus der RCC-26 Linie kombiniert wird. Diese gepoolte cRNA wird dann zum Pulsen von allogenen (HLA-A*0201-positiven) dendritischen Zellen und zur Beurteilung verwendet, ob diese dendritischen Zellen noch zur Aktivierung sowohl des Melanom-spezifischen CTL-Klones, nämlich Tyr F8, als auch des RCC-spezifischen Klones, nämlich TIL-26, in der Lage sind. Wenn dieses Experiment zeigt, daß gepoolte cRNA (in einem Verhältnis von 1:1 aus den beiden Tumor-cRNAs gemischt) zur Aktivierung beider T-Zell-Klone funktioniert, dann können Variationen im Verhältnis der beiden cRNAs verwendet werden, um den Grad zu bestimmen, in dem eine einzelne cRNA-Probe verdünnt werden kann und noch zur DC-Prozessierung bzw. -Verarbeitung und Präsentation funktioniert. Auf Grundlage gepoolter cRNA aus mehreren unterschiedlichen Tumorlinien (beispielsweise drei RCC-Linien) wird dann eine Vakzine erzeugt und diesem Pool wird zusätzliche cRNAs zugesetzt, die aus bekannten, in verschiedenen Nierenzellkarzinomen exprimierten Genen gewonnen werden, die ebenfalls potentielle Zielmoleküle für spezifische CTL's darstellen. Diese könnten HER2/neu, MUC1, PSMA, WT-1, Telomerase und mehrere weitere Kandidaten einschließen (9). Der Einfluß des Zusatzes dieser individuellen cRNA-Spezies kann überprüft werden, indem sie der cRNA von RCC-26 zugesetzt wird und indem die Fähigkeit der gepulsten dendritischen Zellen beurteilt wird, TIL-26-Zellen zu aktivieren.

### Vergleich von autologen versus HLA-haplo-identische dendritische Zellen als Antigen präsentierende Zellen:

Die vorstehend beschriebenen Experimente wurden dargelegt, um die Verwendung von von nicht verwandten Spendern gewonnenen cRNA-gepulsten allogenen dendritischen Zellen zu etablieren, die nur HLA-A*0201-codierte Moleküle mit den reagierenden T-Zellen teilen. Danach besteht der nächste Schritt darin zu zeigen, daß HLA-haplo-identische dendritische Zellen T-Zellen gegen Tumor-assoziierte Epitope primen können, die durch cRNA in die dendritischen Zellen übertragen wurden. Diese Strategie wird gewählt, weil HLA-haplo-identische DC drei Klasse I-Allele (HLA-A, B und C) und drei Klasse II-Allele (HLA-DR, DQ und DP) mit den T-Zellen teilen. Die unterschiedlichen Klasse I-Moleküle können jeweils einen einzigartigen Peptidsatz binden und unterschiedliche CD8+ CTL primen und die drei Klasse II-codierten Moleküle binden ebenfalls jeweils unterschiedliche Peptidsätze, die sich von denjenigen der Klasse I-Moleküle unterscheiden. Diese Klasse II-Peptid-Liganden aktivieren eine andere Art von T-Lymphozyten, die CD4-Zellen, die Zytokine bereitstellen können, die die optimale Entwicklung einer Immunreaktion unterstützen oder direkt als Effektorzellen fungieren (11).

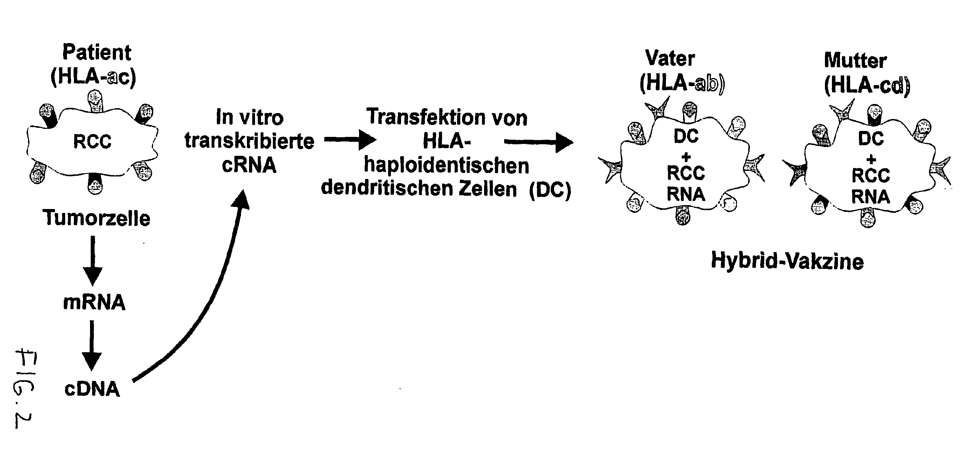

Die Experimente zur Untersuchung der Rolle von HLA-haplo-identischen dendritischen Zellen werden durch Vergleich von autologen dendritischen Zellen, die von normalen Kontroll-Spendern (Spender Nr. 1) präpariert wurden und von HLA-haplo-identischen dendritischen Zellen, die von einem ausgewählten HLA-typisierten Familienmitglied (Spender 2) präpariert wurden, verglichen (d.h. ein Elternteil oder Geschwister wird so ausgewählt, daß er einen HLA-Haplotyp aufweist, den er mit dem Spender 1 teilt und der zweite HLA-Haplotyp diesem nicht entspricht; siehe Abb. 2). Von beiden Spendern präparierte DC's werden mit cRNA von RCC-26 oder der Melanom-Linie gepulst. Die sich ergebenden DC's werden zuerst auf ihre Fähigkeit hin getestet, die Interferon-Gamma Sekretion von Tyr F8 und TIL-26-Zellen wie vorstehend beschrieben zu stimulieren. Im nächsten Schritt werden PBL von Spender Nr. 1 in vitro mit cRNA-gepulsten autologen dendritischen Zellen oder den HLA-haplo-identischen dendritischen Zellen von Spender 2 stimuliert. Im Anschluß an zwei oder mehrere Runden der Restimulation wird das Vorhandensein von T-Zellen, die durch unterschiedliche MHC-Moleküle (sowohl Klasse I als auch Klasse II) präsentierte Epitope erkennen, durch Messen ihrer Interferon-Gamma-Sekretion im Anschluß an die Stimulierung mit cRNA-gepulsten autologen dendritischen Zellen in Abwesenheit und Anwesenheit spezifischer monoklonaler Antikörper bestimmt. Die anti-MHC Klasse I-spezifischen monoklonalen Antikörper (W6/32) werden Wechselwirkungen von CD8⁺ CTL mit den dendritischen Stellenzellen blockieren. Eine verminderte Interferon-Gamma-Sekretion in Gegenwart monoklonaler Antikörper, die für HLA-A, B oder C Moleküle spezifisch sind, zeigt den Grad an, in dem CTL's, die ihre Peptide in Verbindung mit unterschiedlichen Klasse I-Molekülen erkennen, vorliegen. Das Vorliegen von Klasse II-restringierten CD4⁺-Zellen kann durch denselben Ansatz beleuchtet werden, indem Klasse II-spezifische monoklonale Antikörper verwendet werden, die mit HLA-DR, DQ und DP-Moleklülen reagieren (17).

Die erfindungsgemäße Erzeugung von cRNA wird durch das nachfolgende Flußdiagramm veranschaulicht:

### Flußdiagramm zur Herstellung von cRNA

- 1.: Züchten von Tumorzell-Linien in vitro (beispielsweise Melanomlinie 9304.A12 und Nierenzellkarzinomlinie, RCC-26)
- 2.: Extrahieren von RNA aus den Tumorzell-Linien (beispielsweise unter Verwendung von Quigen "RNAEasy" Kit Nr. 75412)
- 3.: Durchführung einer Erststrang-cDNA-Synthese (beispielsweise unter Verwendung von Clontech "Smart PCR" cDNA-Synthese Kit Nr. K1052)
- 4.: Durchführung einer cDNA Amplifikation (beispielsweise unter Verwendung von Clontech "Smart PCR" cDNA-Synthese Kit Nr. K1052)
- 5.: Durchführung einer in vitro Transkription von cDNA (beispielsweise unter Verwendung des "mMessage-mMachine" Kit Nr. 1344 von Ambion)
- 6.: Reinigen von cRNA (beispielsweise unter Verwendung des Quigen "RNAEasy" Kit Nr. 75142)
- 7.: Testen der Integrität der cRNA (beispielsweise unter Verwendung eines Perkin Elmer "One Step RT/PCR" Kits Nr. P/N 4308206)

### Literatur:

1. Childs, R.W. Chernoff, A., Contentin, N., Bahceci, E., Schrump, D., Leitman, S., Read, E.J., Tisdale, J., Dunbar, C., Linehan, W.M., Young, N.S. and Barrett, A.J. 2000. Regression of metastiatic renal-cell carcinoma after nonmyeloablative allogeneic peripheral-blood stem-cell transplanation. N. Engl. J. Med. 14:750-758.
2. Kolb, H.-J., Schattenberg., A., Goldman, J.M., Hertenstein, B., Jacobsen, N., Arcese, W., Ljungman, P., Ferrant, A., Verdonck, L., Niederwieder, D., van Rhee, F., Mittermüller, J., de Witte, T., Holler, E. and Ansara, H. for the European Group for Blood and Marrow Transplantation Working Party Chronic Leukemia. 1995. Graft-versusleukemia effect of Spender lymphocyte transfusions in marrow grafted patients. Blood 86: 2041-2050.
3. Kugler A., Stuhler, G., Walden, P., Zöller, G., Zobywalski, A., Brossart, P., Trefzer, U., Ullrich, S., Müller, C.A., Becker, V., Gross, A.J., Hemmerlein, B., Kanz, L., Müller, G.A., and Ringert, R.-H. 2000. Regression of human metastatic renal cell carcinoma after vaccination with tumor cell-dendritic cell hybrids. Nature Med. 6: 332-336.
4. Kufe, D. 2000. Smallpox. polio and now a cancer vaccine? Nature Med. 6: 252-253.
5. Boczkowski, D., Nair, s.K, Nam, J.H., Lyerly, H.K. and Golboa, E. 2000. Induction of tumor immunity and cytotoxic T lymphocyte responses using dendritic cells transfected with messenger RNA amplified from tumor cells. Cancer Res. 60: 10281034.
6. Heiser, A., Dahm, P., Yancey, D., Maurice, M.A., Boczkowski, D., Nair, S.K., Gilboa, E. and Vieweg, J. 2000. Human dendritic cells transfected with RNA encoding prostate-specific antigen stimulate prostate-specific CTL responses in vitro. J. Immunol. 164: 5508-5014.
7. Nair, S.K., Boczkowski, D., Morse, M., Cumming, R.I., Lyerly, H.K. and Gilboa, E. 1998. Induction of primary carcinoembryonic antigen (CEA)-specific cytotoxic T lymphocytes in vitro using human dendritic cells transfected with RNA. Nat. Biotechnol 16: 364-369.
8. Nair, SK. Hull, S., Coleman, D., Gilboa, E., Lyerly, H.K. and Morse, M.A. 1999. Induction of carcinoembryonic antigen (CEA)-specific cytotoxic T-lymphocyte responses in vitro using autologous dendritic cells loaded with CEA peptide or CEA RNA in patients with metastatic malignancies expressing CEA. Int. J. Cancer 82: 121-124.
9. Dannull, J., Cemy, T., Ackermann, D.K. and Groettrup, M. 2000. Current status of dendritic cell-based tumor vaccination. Onkologie 23:544-551.
10. Visseren, M.J., van Elsas, A., van der Voort. E.l.H., Ressing, M.B., Käst. W.M., Schrier, P.1. and C.J.M. Melief. 1995. CTL specific for the tyrosinase autoantigen can be induced from healthy Spender blood to lyse melanoma cells. J. Immunol. 154:3991-3998.
11. Su, Z., Peluso. M.V.. Raffegerst, S.H,, Schendel, D.J,, and M.A. Roskrow. 2001. LMP2a-specific cytotoxic T lymphocytes for the treatment of patients with relapsed EBV-positive Hodgkin disease. Eur. J. Immunol. 31:947-958.
12. Su, Z., Peluso, M.V., Raffergerst, S.U., Schendel, D.J. and Roskrow, M. Antigen presenting cells transfected with LMP2a RNA induce CD4+ LMP2a-specific cytotoxic T lymphocytes which kill via a Fas-independent mechanism (submitted).
13. Schendel. D.J., Gansbacher. B, Oberneder. R, Kriegmair, M., Hofstetter, A., Riethmüller, G. and O.G. Segurado. 1993. Tumor-specific lysis of human renal cell carcinomas by tumor-infiltrating lymphocytes.1. HLA-A2 restricted lysis of autologous and aliogeneic tumor lines. J. Immunol, 151:4209-4220.
14. Schendel, D J., Frankenberger, B., Jantzer, P, Cayeux, S., Nößner, E., Willimsky, G., Maget, B., Pohla. H., and Th. Blankenstein. 2000. Expression of B7.1 (CD80) in a renal cell carcinoma line allows expansion of tumor-associated cytotoxic T lymphocytes in the presence of an alloresponse. Gene Therapy 7: 2007-2014.
15. Jantzer, P. and D.J. Schendel. 1998. Human renal cell carcinoma antigen-specific cytotoxic T lymphocytes: antigen-driven selection and long-term persistence in vivo. Cancer Res. 58: 3078-3086.
16. Altman. J.D., Moss. P.A., Goulder, P.J., Barouch, D.H., McHeyzer-Willliams, M.G., Bell, J. 1., McMichael. A.J. and Davis, M.M. 1996. Phenotypic analysis of antigenspecific T Lymphocytes. Science 274: 94-96.
17. Gong, J., Nikrui, N., Chen, D., Koido, S., Wu, Z., Tanaka, Y., Cannistra, S., Avigan, D. and Kufe, D. 2000. Fusions of human ovarian carcinoma cells with autologous or allogeneic dendritic cells induce antitumor immunity. J. Immunol. 165: 1705-1711.

## Patentansprüche

1. Verfahren zur Erzeugung von HLA-haploidentischen Antigen-präsentierenden Zellen zur Behandlung von Tumorerkrankungen bei einem Patienten, das die folgenden Schritte umfasst:
- Bereitstellen von Antigen-präsentierenden Zellen von einem Spender, die bezüglich denen des Patienten HLA-haploidentisch sind;
- Einbringen von Proteinen und/oder Peptiden oder RNA oder DNA oder cDNA, die für diese Proteine und/oder Peptide codieren, die in Tumorzellen überexprimiert werden oder die aus autologen Tumorzellen stammen, in die HLA-haploidentischen Antigen-präsentierenden Zellen.

2. Verfahren nach Anspruch 1, bei dem Proteine und/oder Peptide oder RNA bzw. DNA oder cDNA, die für diese Proteine und/oder Peptide codieren, aus mehreren unterschiedlichen Tumorzell-Linien in die HLA-haploidentischen Antigen-präsentierenden Zellen eingebracht werden.

3. Verfahren nach Anspruch 1 oder 2, **dadurch gekennzeichnet,**
**daß** zunächst RNA aus Tumorzellen in cDNA rücktranskribiert, die cDNA mit Hilfe der PCR amplifiziert und anschließend die cDNA in RNA transkribiert wird.

4. Verfahren nach einem der Ansprüche 1-3, bei dem Antigen-präsentierenden Zellen von zwei unterschiedlichen HLA-haploidentischen Individuen eingesetzt werden.

5. HLA-haploidentische Antigen-präsentierende Zellen, die durch eines der Verfahren nach Anspruch 1 - 4 gewinnbar sind.

6. HLA-haploidentische Antigen-präsentierende Zellen nach Anspruch 5,
**dadurch gekennzeichnet,**
**daß** die Proteine und/oder Peptide oder RNA oder DNA oder cDNA, die für diese Proteine und/oder Peptide codieren, die in Tumorzellen überexprimiert werden oder die aus autologen Tumorzellen stammen, ausgewählt werden aus folgenden Tumorzellen: Karzinomen, bevorzugt Ovarial-, Mamma- und Nierenzellkarzinomen, Tumorzellen des blutbildenden Systems, bevorzugt Zellen aus Leukämien und Lymphomen, Zellen aus mesenchymalen Tumoren, bevorzugt Sarkomen, Zellen aus epithelealen Tumoren, Zellen aus ektodermalen Tumoren, bevorzugt Melanomen, und Zellen aus embryonalen Tumoren aus undifferenziertem Gewebe, bevorzugt Blastomen und Teratomen.

7. HLA-haploidentische Antigen-präsentierende Zellen nach Anspruch 5 oder 6, die Proteine und/oder Peptide oder RNA oder DNA oder cDNA, die für diese Proteine und/oder Peptide codieren, aus mehreren unterschiedlichen Tumorzell-Linien enthalten.

8. HLA-haploidentische Antigen-präsentierende Zellen nach Anspruch 5 - 7,
**dadurch gekennzeichnet,**
**daß** die Antigen-präsentierenden Zellen dendritische Zellen oder Makrophagen sind.

9. Pharmazeutische Zusammensetzung, enthaltend HLA-haploidentische Antigen-präsentierende Zellen nach einem der Ansprüche 5 - 8.

10. Zusammensetzung nach Anspruch 9,
**dadurch gekennzeichnet,**
**daß** es sich um eine Vakzine handelt.

11. Verwendung HLA-haploidentischer Antigen-präsentierender Zellen nach einem der Ansprüche 5 - 8 zur Herstellung eines Medikamentes zur Behandlung von Tumorerkrankungen bei dem Patienten.

12. Verwendung nach Anspruch 11,
**dadurch gekennzeichnet,**
**daß** die Tumorzellen umfassen: Zellen aus Karzinomen, bevorzugt Ovarial-, Mamma- und Nierenzellkarzinomen, Tumorzellen des blutbildenden Systems, bevorzugt Zellen aus Leukämien und Lymphomen, Zellen aus mesenchymalen Tumoren, bevorzugt Sarkomen, Zellen aus epithelealen Tumoren, Zellen aus ektodermalen Tumoren, bevorzugt Melanomen, und Zellen aus embryonalen Tumoren aus undifferenziertem Gewebe, bevorzugt Blastomen und Teratomen.

13. Verwendung nach Anspruch 12,
**dadurch gekennzeichnet,**
**daß** die HLA-haploidentischen Antigen-präsentierenden Zellen zur Behandlung von Tumoren eingesetzt werden, umfassend: Karzinome, bevorzugt Ovarial-, Mamma- und Nierenzellkarzinome, Tumore des blutbildenden Systems, bevorzugt Leukämien und Lymphome, mesenchymale Tumore, bevorzugt Sarkome, epitheliale Tumore, ektodermale Tumore, bevorzugt Melanome, und embryonale Tumore aus undifferenziertem Gewebe, bevorzugt Blastome und Teratome.

14. Verwendung nach einem oder mehreren der Ansprüche 11-13,
**dadurch gekennzeichnet,**
**daß** HLA-haploidentische Antigen-präsentierende Zellen von zwei unterschiedlichen HLA-haploidentischen Individuen eingesetzt werden.

15. Verwendung nach Anspruch 14,
**dadurch gekennzeichnet,**
**daß** RNA eingesetzt wird, die aus autologen Tumorzellen in cDNA rücktranskribiert, diese cDNA mit Hilfe der PCR amplifiziert und anschließend die cDNA in RNA transkribiert wurde.

16. Verwendung nach einem oder mehreren der Ansprüche 11-15,
**dadurch gekennzeichnet,**
**daß** die HLA-haploidentischen Antigen-präsentierenden Zellen zur intravenösen, subkutanen oder intramuskulären Applikation vorgesehen sind.

17. Verwendung von HLA-haploidentischen Antigen-präsentierenden Zellen nach einem der Ansprüche 11-16, in welchen Proteine und/oder Peptide oder RNA bzw. DNA oder cDNA, die für diese Proteine und/oder Peptide codieren, die in Tumorzellen überexprimiert werden oder die aus autologen Tumorzellen stammen, in rekombinanter Form eingebracht sind.

18. Verwendung nach einem oder mehreren der Ansprüche 11-17,
**dadurch gekennzeichnet,**
**daß** RNA oder DNA oder cDNA in die HLA-haploidentischen Antigen-präsentierenden Zellen eingebracht ist, die für tumordefinierte Antigene codiert, die tumordefinierten Antigene in den Tumorzellen überexprimierte Antigene sind, und bevorzugt ausgewählt werden aus Onkogenen, bevorzugt HER2/neu, Proteinen, die dem Tumor einen Wachstumsvorteil verschaffen und/oder sein Überleben sichern, bevorzugt PSMA, Zellzyklusregulationsproteinen, Transkriptionsfaktoren, bevorzugt WT-1, Mucinen, bevorzugt MUC1, Proteinen, die in der Regulation der Zellteilung involviert sind, bevorzugt Telomerase.

19. Verwendung nach einem oder mehreren der Ansprüche 11-18,
**dadurch gekennzeichnet,**
**daß** die Antigen-präsentierenden Zellen dendritische Zellen oder Makrophagen sind.

20. Verwendung HLA-haploidentischer Antigen-präsentierender Zellen nach einem oder mehreren der Ansprüche 11-19, in welche Proteine und/oder Peptide oder RNA oder DNA oder cDNA, die für diese Proteine und/oder Peptide codieren, aus mehreren unterschiedlichen Tumorzell-Linien eingebracht sind, zur Herstellung eines Medikamentes zur Behandlung von Tumorerkrankungen bei dem Patienten.

21. Verwendung nach Anspruch 20, bei der gepoolte cRNA aus zwei oder drei unterschiedlichen Tumorzelllinien eingebracht wird.

## Claims

1. A method for the generation of HLA-haploidentical antigen-presenting cells for the treatment of tumor diseases in a patient comprising the following steps:
- providing antigen-presenting cells from a donor which are HLA-haploidentical with respect to those of the patient;
- introducing proteins and/or peptides or RNA or DNA or cDNA encoding said proteins and/or peptides which are overexpressed in tumor cells or are derived from autologous tumor cells are introduced into the HLA-haploidentical antigen-presenting cells.

2. The method according to claim 1 wherein proteins and/or peptides or RNA or DNA or cDNA, respectively, encoding said proteins and/or peptides from several different tumor cell lines are introduced into the HLA-haploidentical antigen-presenting cells.

3. The method according to claim 1 or 2 **characterized in that** first RNA from tumor cells is reverse transcribed into cDNA, the cDNA is amplified by means of PCR and subsequently the cDNA is transcribed into RNA.

4. The method according to anyone of claims 1-3 wherein antigen-presenting cells of two different HLA-haploidentical individuals are used.

5. The HLA-haploidentical antigen-presenting cells obtained by a method according to claim 1-4.

6. The HLA-haploidentical antigen-presenting cells according to claim 5 **characterized in that** said proteins and/or peptides or RNA or DNA or cDNA encoding said proteins and/or peptides which are overexpressed in tumor cells or are derived from autologous tumor cells are selected from the following tumor cells: carcinomas, preferably ovarian, mammary and renal cell carcinomas, tumor cells of the hematopoietic system, preferably cells of leukemias and lymphomas, cells of mesenchymal tumors, preferably sarcomas, cells of epithelial tumors, cells of ectodermal tumors, preferably melanomas, and cells of embryonic tumors from undifferentiated tissue, preferably blastomas and teratomas.

7. The HLA-haploidentical antigen-presenting cells according to claim 5 or 6 containing proteins and/or peptides or RNA or DNA or cDNA encoding said proteins and/or peptides from several different tumor cell lines.

8. The HLA-haploidentical antigen-presenting cells according to claims 5-7 **characterized in that** said antigen-presenting cells are dendritic cells or macrophages.

9. A pharmaceutical composition containing HLA-haploidentical antigen-presenting cells according to anyone of claims 5-8.

10. A composition according to claim 9 **characterized in that** it is a vaccine.

11. Use of HLA-haploidentical antigen-presenting cells according to anyone of claims 5-8 for the preparation of a medicament for the treatment of tumor diseases in a patient.

12. The use according to claim 11 **characterized in that** said tumor cells comprise: cells of carcinomas, preferably ovarian, mammary and renal cell carcinomas, tumor cells of the hematopoietic system, preferably cells of leukemias and lymphomas, cells of mesenchymal tumors, preferably sarcomas, cells of epithelial tumors, cells of ectodermal tumors, preferably melanomas, and cells of embryonic tumors from undifferentiated tissue, preferably blastomas and teratomas.

13. The use according to claim 12 **characterized in that** said HLA-haploidentical antigen-presenting cells are used for the treatment of tumors comprising: carcinomas, preferably ovarian, mammary and renal cell carcinomas, tumors of the hematopoietic system, preferably leukemias and lymphomas, mesenchymal tumors, preferably sarcomas, epithelial tumors, ectodermal tumors, preferably melanomas, and embryonic tumors from undifferentiated tissue, preferably blastomas and teratomas.

14. The use according to one or more of claims 11-13 **characterized in that** HLA-haploidentical antigen-presenting cells of two different HLA-haploidentical individuals are used.

15. The use according to claim 14 **characterized in that** RNA is employed which has been reverse transcribed from autologous tumor cells into cDNA, the cDNA has been amplified by means of PCR and subsequently the cDNA has been transcribed in RNA.

16. The use according to one or more of claims 11-15 **characterized in that** said HLA-haploidentical antigen-presenting cells are intended for intravenous, subcutaneous or intramuscular application.

17. The use of said HLA-haploidentical antigen-presenting cells according to anyone of claims 11-16 into which proteins and/or peptides or RNA or DNA or cDNA, respectively, encoding said proteins and/or peptides which are overexpressed in tumor cells or are derived from autologous tumor cells have been introduced in recombinant form.

18. The use according to one or more of claims 11-17 **characterized in that** RNA or DNA or cDNA is introduced into the HLA-haploidentical antigen-presenting cells which encodes tumor-defined antigens, wherein the tumor-defined antigens are antigens overexpressed in the tumor cells and are preferably selected from oncogenes, preferably HER2/neu, proteins providing a growth advantage to the tumor and/or ensuring its survival, preferably PSMA, cell cycle regulatory proteins, transcription factors, preferably WT-1, mucins, preferably MUC-1, proteins involved in the regulation of cell division, preferably telomerase.

19. The use according to one or more of claims 11-18 **characterized in that** said antigen-presenting cells are dendritic cells or macrophages.

20. The use of HLA-haploidentical antigen-presenting cells according to one or more of claims 11-19, into which proteins and/or peptides or RNA or DNA or cDNA encoding said proteins and/or peptides from several different tumor cell lines have been introduced for the manufacture of a medicament for treatment of tumor diseases in said patient.

21. The use according to claim 20 wherein pooled cRNA from two or three different tumor cell lines is introduced.

## Revendications

1. Procédé de production de cellules présentant l'antigène haploidentique HLA servant au traitement des maladies tumorales chez un patient qui comprend les étapes suivantes :
- mise à disposition de cellules d'un donneur présentant l'antigène haploidentique HLA à celles du patient ;
- apport de protéines et/ou de peptides ou d'ARN, d'ADN ou d'ADNc codant pour ces protéines et/ou peptides, qui sont surexprimées dans des cellules tumorales ou qui proviennent de cellules tumorales autologues, dans les cellules présentant l'antigène haploidentique HLA.

2. Procédé selon la revendication 1, dans lequel des protéines et/ou des peptides, l'ARN et/ou l'ADN ou l'ADNc codant pour ces protéines et/ou peptides, sont introduites à partir de plusieurs lignées de cellules tumorales différentes dans les cellules présentant l'antigène haploidentique HLA.

3. Procédé selon la revendication 1 ou 2, **caractérisé en ce que** l'ARN est d'abord retranscrit à partir des cellules tumorales dans l'ADNc, l'ADN est amplifié à l'aide du PCR et transcrit ensuite l'ADNc dans l'ARN.

4. Procédé selon l'une quelconque des revendications 1 à 3, dans lequel des cellules présentant l'antigène de deux individus différents haploidentiques HLA sont utilisées.

5. Cellules présentant l'antigène haploidentique HLA susceptibles d'être obtenues par un procédé selon l'une quelconque des revendications 1 à 4.

6. Cellules présentant l'antigène haploidentique HLA selon la revendication 5, **caractérisées en ce que** les protéines et/ou les peptides ou l'ARN ou l'ADN ou l'ADNc codant pour ces protéines et/ou peptides qui sont surexprimées dans les cellules tumorales ou qui proviennent de cellules tumorales autologues, sont choisis parmi les cellules tumorales suivantes : des carcinomes, de préférence des carcinomes rénaux, mammaires et ovariens, cellules tumorales du système hématopoïétique, de préférence des cellules de leucémies et de lymphomes, des cellules de tumeurs mésenchymales, de préférence des sarcomes, des cellules de tumeurs épithéliales, des cellules de tumeurs ectodermiques, de préférence des mélanomes et des cellules de tumeurs embryonnaires du tissu indifférencié, de préférence des blastomes et des tératomes.

7. Cellules présentant l'antigène haploidentique HLA selon la revendication 5 ou 6, qui contiennent des protéines et/ou peptides ou de l'ARN ou de l'ADN ou de l'ADNc codant pour ces protéines et/ou peptides, à partir de plusieurs lignées de cellules tumorales différentes.

8. Cellules présentant l'antigène haploidentique HLA selon l'une quelconque des revendications 5 à 7, **caractérisées en ce que** les cellules présentant l'antigène sont des cellules dendritiques ou macrophages.

9. Composition pharmaceutique contenant des cellules présentant l'antigène haploidentique HLA selon l'une quelconque des revendications 5 à 8.

10. Composition selon la revendication 9, **caractérisée en ce qu'**il s'agit d'un vaccin.

11. Utilisation de cellules présentant l'antigène haploidentique HLA selon l'une quelconque des revendications 5 à 8 pour la fabrication d'un médicament servant au traitement des maladies tumorales chez le patient.

12. Utilisation selon la revendication 11, **caractérisée en ce que** les cellules tumorales comprennent : des cellules de carcinomes, de préférence des carcinomes rénaux, mammaires et ovariens, des cellules tumorales du système hématopoïétique, de préférence des cellules de leucémies et de lymphomes, des cellules de tumeurs mésenchymales, de préférence des sarcomes, des cellules de tumeurs épithéliales, des cellules de tumeurs ectodermiques, de préférence des mélanomes et des cellules de tumeurs embryonnaires du tissu indifférencié, de préférence des blastomes et des tératomes.

13. Utilisation selon la revendication 12, **caractérisée en ce que** les cellules présentant l'antigène haploidentique HLA sont utilisées pour le traitement des tumeurs comprenant : des carcinomes, de préférence des carcinomes rénaux, mammaires et ovariens, des tumeurs du système hématopoïétique, de préférence des de leucémies et de lymphomes, des tumeurs mésenchymales, de préférence des sarcomes, des tumeurs épithéliales, des tumeurs ectodermiques, de préférence des mélanomes et des tumeurs embryonnaires du tissu indifférencié, de préférence des blastomes et des tératomes.

14. Utilisation selon une un ou plusieurs des revendications 11 à 13, **caractérisée en ce que** des cellules présentant l'antigène haploidentique HLA de deux individus différents haploidentiques HLA sont utilisées.

15. Utilisation selon la revendication 14, **caractérisée en ce que** de l'ARN est utilisée qui a été retranscrit à partir de cellules tumorales autologues dans l'ADNc, cet ADNc ayant été amplifié par PCR et l'ADNc ayant été transcrit ensuite en ARN.

16. Utilisation selon une ou plusieurs des revendications 11 à 15, **caractérisée en ce que** les cellules présentant l'antigène haploidentique HLA sont prévues pour une application intraveineuse, subcutanée ou intramusculaire.

17. Utilisation de cellules présentant l'antigène haploidentique HLA selon l'une quelconque des revendications 11 à 16, dans laquelle les protéines et/ou peptides ou l'ARN et/ou l'ADN ou l'ADNc codant pour ces protéines et/ou peptides qui sont surexprimées dans des cellules tumorales ou qui proviennent de cellules tumorales autologues, sont introduites sous une forme recombinée.

18. Utilisation selon une ou plusieurs des revendications 11 à 17, **caractérisée en ce que** l'ARN ou l'ADN ou l'ADNc apportés dans les cellules présentant l'antigène haploidentique HLA codant pour des antigènes tumoraux, les antigènes tumoraux dans les cellules tumorales sont des antigènes surexprimés, et sont choisis de préférence parmi des oncogènes, de préférence HER2/neu, des protéines qui procurent à la tumeur un avantage de croissance et/ou assurent sa survie, de préférence des PSMA, des protéines de régulation du cycle cellulaire, des facteurs de transcription, de préférence WT-1, des mucines, de préférence MUC1, des protéines qui sont impliquées dans la régulation de la division cellulaire, de préférence la télomérase.

19. Utilisation selon une ou plusieurs des revendications 11 à 18, **caractérisée en ce que** les cellules présentant l'antigène sont des cellules dendritiques ou macrophages.

20. Utilisation de cellules présentant l'antigène haploidentique HLA selon une ou plusieurs revendications 11 à 19, dans laquelle sont introduites des protéines et/ou peptides ou ARN ou ADN ou ADNc codant pour ces protéines et/ou peptides provenant de plusieurs lignées de tumeurs cellulaires différentes pour la production d'un médicament servant au traitement des maladies tumorales chez le patient.

21. Utilisation selon la revendication 20 dans laquelle est introduit de l'ARNc réuni provenant de deux ou trois lignées de cellules tumorales différentes.
